# DEMANDE DE BREVET EUROPEEN

(11) **EP 2 431 479 A2**
(43) Date de publication de la demande: **21.03.2012**
(21) Numéro de dépôt: 11189033.1
(22) Date de dépôt: 09.02.2006
(51) Int. Cl.: C12Q 1/04

(54) **Milieux pour la détection spécifique de microorganismes résistants**

(30) Priorité: 10.02.2005 FR 0550394; 07.10.2005 FR 0553049
(62) Demande divisionnaire de: 06709488.8
(71) Demandeur: Biomérieux, 69280 Marcy L'Etoile (FR)
(72) Inventeur: Orenga, Sylvain, 01160 Neuville Sur Ain (FR); Roger-Dalbert, Céline, 01150 Chazey Sur Ain (FR); Perry, John, Newcastle upon Tyne, NE7 7BH (GB); Chanteperdrix, Vanessa, 38000 Grenoble (FR); Zambardi, Gilles, 38300 Chezeneuve (FR); Bal, Nathalie, 69140 Rillieux la Pape (FR)
(74) Mandataire: Bitaud, Valérie Marie-Odile

(57) **Abrégé**

L'invention concerne l'utilisation d'un milieu de culture pour distinguer au moins trois groupes de microorganismes dans un échantillon biologique, ledit échantillon comprenant :
● un premier groupe de levures, appartenant à un premier taxon de levures et comprenant au moins un mécanisme de résistance à un traitement ;
● un deuxième groupe de levures, appartenant à un deuxième taxon de levures, différent dudit premier taxon, mais comprenant au moins un mécanisme de résistance au même traitement ;
● un troisième groupe de levures non résistantes audit traitement ;

ledit milieu de culture comprenant :
a) au moins un premier substrat permettant la détection d'au moins une première activité enzymatique ou métabolique dudit premier groupe de levures ;
b) au moins un marqueur permettant la différenciation du premier groupe de microorganismes et du deuxième groupe de microorganismes, ledit marqueur étant un substrat permettant la détection d'au moins un activité enzymatique ou métabolique dudit deuxième groupe de levures ;
c) au moins un antimicrobien, actif sur ledit troisième groupe de levures.

## Description

Le domaine de l'invention est celui de l'analyse microbiologique par voie biochimique, et en particulier de la détection et de l'identification de microorganismes, tels que notamment des levures.

La résistance des bactéries aux antibiotiques est un problème majeur de santé publique. La résistance de microorganismes infectieux envers un traitement s'est développée en même temps que les molécules anti-infectieuses et représente aujourd'hui un obstacle majeur en thérapeutique. Cette résistance est source de problèmes multiples, incluant les difficultés de détection au laboratoire, les options de traitement limitées et un impact délétère sur l'issue clinique.

En particulier, l'augmentation rapide et irrépressible de la résistance des bactéries pathogènes, au cours des 20 dernières années, représente un des problèmes actuels majeurs de la médecine. Les infections causées par ces organismes sont à l'origine de l'allongement de la durée d'hospitalisation et sont associées à des taux élevés de morbidité et de mortalité, suite à des échecs thérapeutiques.

Plusieurs mécanismes de résistance peuvent être impliqués simultanément chez une souche bactérienne. Ils se classent en général en 3 catégories : défaut de pénétration de l'antibiotique dans la bactérie, inactivation ou excrétion de l'antibiotique par des systèmes enzymatiques bactériens et défaut d'affinité entre la cible bactérienne et l'antibiotique.

L'inactivation enzymatique est le mécanisme le plus fréquent de résistance acquise en termes de nombre d'espèces et d'antibiotiques concernés. Ainsi, les céphalosporinases chromosomiques de classe C constituent aujourd'hui un des mécanismes de résistance prédominants des bactéries gram-négatif, les bactéries exprimant de telles enzymes étant résistantes aux céphalosporines. De même, les β-lactamases sont des enzymes exprimées par certaines bactéries, capables d'hydrolyser la liaison C-N du noyau β-lactame, structure de base des antibiotiques de la famille des β-lactamines, pour donner un produit microbiologiquement inactif. Plusieurs inhibiteurs de β-lactamases (IBL), tels que l'acide clavulanique (AC), le tazobactam et le sulbactam, ont été développés pour augmenter l'activité antimicrobienne et élargir le spectre des β-lactamines qui leur sont associées. Agissant comme un substrat suicide des β-lactamases, ils empêchent la dégradation enzymatique des antibiotiques et leur permettent de devenir efficaces contre des bactéries initialement résistantes. Cependant, de par l'exposition persistante des souches à la pression antibiotique, les bactéries expriment leur capacité d'adaptation par la production continue et dynamique de β-lactamases, évoluant en même temps que le développement de nouvelles molécules.

Les bactéries gram-négatif productrices de céphalosporinases chromosomiques de classe C de haut niveau (on parle de bactéries Case HN), ainsi que les bactéries gram négatif productrices de β-lactamases à spectre étendu (on parle alors de bactéries BLSE) sont devenues de ce fait une menace grandissante, en particulier parce que le nombre d'espèces bactériennes concernées augmente. Les bactéries Case HN et BLSE sont résistantes à des traitements à base de pénicillines et céphalosporines de 1ère et 2ème générations, mais également de céphalosporines de 3ème génération (C3G) (cefotaxime CTX, ceftazidime CAZ, cefpodoxime CPD, ceftriaxone CRO) et des monobactams (aztreonam ATM). Par contre, les 7α-méthoxycéphalosporines (céphamycines: cefoxitine, cefotetan) et les carbapénèmes (imipénème, méropénème, ertapénème) conservent généralement leur activité. Les BLSE sont inhibées par les inhibiteurs de β-lactamases (IBL), ce qui permet de les différencier des autres céphalosporinases.

Ces bactéries expriment ainsi le plus souvent simultanément des résistances à plusieurs traitements, ce qui pose des difficultés pour installer un traitement pertinent et éviter les échecs thérapeutiques. Une bactérie *Escherichia coli* peut ainsi être Case HN et BLSE. En outre, comme les entérobactéries BLSE positives ont tendance à disséminer la résistance par transmission clonale de souches ou transfert plasmidique conjugatif, elles représentent un problème pour le contrôle des infections. Dans la plupart des études, *Escherichia coli* et *Klebsiella pneumoniae* demeurent les espèces productrices de BLSE les plus communes. Mais les BLSE ont, depuis quelques années, grandement élargi leur panel d'espèces hôtes. En effet, de nombreuses espèces d'entérobactéries et de bacilles gram-négatif non fermentants (comme *Pseudomonas aeruginosa*) ont également été rapportés comme producteurs de BLSE.

Outre ces bactéries BLSE, on peut également citer les bactéries *Staphylococcus aureus* qui sont également des bactéries pathogènes développant de nombreux mécanismes de résistances, telles qu'une résistance à la méticilline, la pénicilline, la tétracycline, l'érythromycine, la vancomycine. *Enterococcus faecium* est une autre bactérie multirésistante trouvée en milieu hospitalier, qui peut être résistante à la pénicilline, la vancomycine et au linezolide. *Mycobacterium tuberculosis* est couramment résistant à l'isoniazide et au rifampicine. D'autres pathogènes offrent certaines résistances comme *Salmonella, Campylobacter* et *Streptococcus.*

Il devient donc indispensable, d'un point de vue de santé publique, de pouvoir identifier le plus rapidement possible de tels microorganismes, et de tels mécanismes de résistances.

En général, la recherche des micro-organismes résistants à un traitement se fait selon les étapes suivantes
1. prélèvement d'un échantillon biologique susceptible de contenir lesdits micro-organismes
2. ensemencement et incubation d'un milieu de culture (18 à 48H) pour induire une croissance exponentielle des micro-organismes
3. Repérage sur les milieux de culture des colonies de micro-organismes potentiellement significatifs
4. Caractérisation de l'espèce de micro-organisme
5. Identification des mécanismes de résistance des micro-organismes analysés, leur signification biologique et éventuellement la thérapie adaptée.

Cette succession d'étapes implique un temps important entre le prélèvement de l'échantillon susceptible de contenir des micro-organismes, et la prescription d'un traitement adapté pour le patient. De plus, l'utilisateur doit généralement réaliser manuellement des étapes de transfert de micro-organismes d'un premier milieu vers un deuxième milieu, ce qui peut induire des problèmes notamment de contamination mais également des risques pour la santé du manipulateur.

A titre d'exemple, pour détecter la présence de béta-lactamases à spectre étendu (BLSE) dans des souches *d'Escherichia coli* et de *Klebsiella pneumoniae,* on peut utiliser une technique de diffusion telle que décrite dans la publication de Jacoby & Han (J Clin Microbiol. 34(4): 908-11, 1996) qui ne donne toutefois aucune information sur l'identification des souches testées : on peut déterminer si la bactéries est une bactérie productrice de BLSE ou non, mais on ne peut distinguer si une telle bactérie est une *Escherichia coli* ou une *Klebsiella pneumoniae.* Des substrats métaboliques sont également utilisés pour détecter la présence de BLSE ou Case HN. A ce titre, les laboratoires AES proposent un milieu dans une bi boîte associant un milieu Drigalski avec du céfotaxime et un milieu MacConkey avec de la Ceftazidime. Les milieux Drigalski et MacConkey permettent de révéler l'acidification du Lactose, métabolisme présent chez un très grand nombre d'espèces d'entérobactéries. Toutefois, un tel milieu permet uniquement de distinguer des bactéries résistantes de bactéries non résistantes, mais ne permet pas de distinguer les bactéries exprimant une BLSE de celles exprimant une Case HN. Ce milieu ne permet pas non plus l'identification d'espèces particulières de bactéries, et ne permet pas, par exemple de discriminer les bactéries *E. coli,* de bactéries K. *pneumoniae.*

Dans le cas de la détection de mécanismes de résistance autre que BLSE, on peut citer la demande de brevet EP0954560 qui concerne la recherche des entérocoques résistants à la Vancomycine, en combinant la Vancomycine avec un milieu chromogène révélant deux activités enzymatiques (β-glucosidase et pyrrolidonyl-arylamidase). Toutefois, ce milieu chromogène permet de déterminer uniquement si les souches résistantes à la vancomycine appartiennent ou n'appartiennent pas au genre *Enterococcus,* mais ne permet pas d'identifier l'espèce ou les mécanismes de résistance impliqués, notamment s'il s'agit d'une résistance acquise ou sauvage.

Ainsi, la caractérisation d'une espèce de microorganisme, puis l'identification de sa résistance à un traitement, est longue et fastidieuse. Si le laboratoire rend au clinicien un dépistage positif alors que l'isolat est en fait dépourvu de microorganismes résistants, cela peut entraîner un traitement inutile et inadapté. Inversement, ne pas communiquer un dépistage positif qui sera ensuite confirmé retarde la mise en place de l'isolement du patient (et éventuellement d'une thérapeutique adaptée) d'une journée. Cela montre le besoin d'un test de confirmation rapide et fiable.

La présente invention se propose donc d'améliorer l'état de la technique en présentant un nouvel outil de diagnostic permettant un gain de temps, de fiabilité et de pertinence dans la thérapie mise en oeuvre. Notre invention permet en une seule étape d'identifier les espèces de micro-organismes présents dans un échantillon, déterminer leur mécanisme de résistance afin de proposer un traitement adapté à chaque patient. Cette invention est particulièrement adaptée pour discriminer différentes espèces de microorganismes, présentant différents mécanismes de résistance à différent traitement, mais toutes susceptible d'être présentes dans un même échantillon.

Avant d'aller plus avant dans l'exposé de l'invention, les définitions suivantes sont données afin de faciliter la compréhension de l'invention :
Par milieu de culture, on entend un milieu comprenant tous les éléments nécessaires à la survie et/ou à la croissance de microorganismes. Le milieu de culture selon l'invention peut contenir d'éventuels autres additifs comme par exemple: des peptones, un ou plusieurs facteurs de croissance, des hydrates de carbone, un ou plusieurs agents sélectifs, des tampons, un ou plusieurs gélifiants... Ce milieu de culture peut se présenter sous forme de liquide, de gel prêt à l'emploi, c'est à dire prêt à l'ensemencement en tube, flacon, ou sur boite de Petri.
Au sens de la présente invention, le terme microorganisme recouvre les bactéries, gram positif ou gram négatif, les levures et plus généralement, les organismes généralement unicellulaires, invisibles à l'oeil nu, qui peuvent être multipliés et manipulés en laboratoire.
A titre de bactéries Gram négatif, on peut citer les bactéries des genres suivants: *Pseudomonas, Escherichia, Salmonella, Shigella, Enterobacter, Klebsiella, Serratia, Proteus, Campylobacter, Haemophilus, Morganella, Vibrio, Yersinia, Acinetobacter, Branhamella, Neisseria, Burkholderia, Citrobacter, Hafnia, Edwardsiella, Aeromonas, Moraxella, Pasteurella, Providencia, et Legionella.*
A titre de bactéries Gram positif, on peut citer les bactéries des genres suivants: *Enterococcus, Streptococcus, Staphylococcus, Bacillus, Listeria, Clostridium, Gardnerella, Kocuria, Lactococcus, Leuconostoc, Micrococcus, Mycobacteria et Corynebacteria.*
A titre de levures, on peut citer les levures des genres suivants: *Candida, Cryptococcus, Saccharomyces et Trichosporon.*
Par échantillon biologique, on entend un échantillon clinique, issu d'un prélèvement de liquide biologique, ou un échantillon alimentaire, issu de tout type d'aliment. Cet échantillon peut être ainsi liquide ou solide et on peut citer d'une manière non limitative, un échantillon clinique de sang, de plasma, d'urines, de fécès, de prélèvements de nez, de gorges, de peaux, de plaies, de liquide céphalo-rachidien, un échantillon alimentaire d'eau, de boissons tels que le lait, un jus de fruits; de yaourt, de viande, d'oeufs, de légumes, de mayonnaise, de fromage ; de poisson..., un échantillon alimentaire issu d'une alimentation destinée aux animaux, tel que notamment un échantillon issu de farines animales.
Par mécanisme de résistance, on entend tout type de dispositif qui permet à un micro-organisme de rendre un traitement partiellement ou totalement inopérant sur ledit microorganisme, garantissant sa survie. Les mécanismes de résistance se classent en général en 3 catégories : défaut de pénétration de l'antibiotique dans la bactérie, inactivation ou excrétion de l'antibiotique par des systèmes enzymatiques bactériens et défaut d'affinité entre la cible bactérienne et l'antibiotique.
A titre indicatif, on peut citer notamment les mécanismes de résistance liés à l'expression d'une enzyme appartenant au groupe des β-lactamases à spectre étendu ; d'une enzyme appartenant au groupe des céphalosporinases chromosomiques de classe C haut niveau ; les mécanismes de résistance aux glycopeptides, préférentiellement développés par des bactéries appartenant au genre *Enterococcus.*
On citera également les mécanismes de résistance à la méticilline, la pénicilline, la tétracycline, l'érythromycine, la vancomycine lorsque le microorganismes est une bactérie *Staphylococcus aureus*
On citera également les mécanismes de résistance à la pénicilline, la vancomycine et au linezolide lorsque le microorganismes est une bactérie *Enterococcus faecium.*
On citera également les mécanismes de résistance à l'amphotéricine B ou aux antifongiques de la famille des azolés lorsque le microorganisme est une levure.
On citera enfin les mécanismes de résistance à l'isoniazide et au rifampicine lorsque le microorganismes est une bactérie *Mycobacterium tuberculosis.*
Par traitement, on entend un traitement susceptible d'empêcher ou de réduire la croissance de microorganismes issus d'un patient. Ce traitement peut comprendre notamment des composés antimicrobiens, tels que des antibiotiques, tels que pénicillines, céphalosporines classiques, céphalosporines à spectre étendu, monobactams, glycopeptides, aminosides ou tels que des antifongiques ou des composés inhibiteurs de la résistance. A noter que ce traitement peut comprendre en outre l'isolement du patient, ce qui empêche la propagation du microorganisme au sein d'autres patients.
Par substrat permettant la détection d'une activité enzymatique ou métabolique, on entend toute molécule susceptible d'engendrer directement ou indirectement un signal détectable dû à une activité enzymatique ou métabolique du microorganisme.
Lorsque cette activité est une activité enzymatique, on parle alors de substrat enzymatique. Par substrat enzymatique, on entend tout substrat pouvant être hydrolysé par une enzyme en un produit permettant la détection, directe ou indirecte d'un microorganisme. Ce substrat comprend notamment une première partie spécifique de l'activité enzymatique à révéler et une seconde partie faisant office de marqueur, ci-après appelée partie marqueur. Cette partie marqueur peut être chromogène, fluorogène, luminescente... Comme substrat chromogène, bien adapté aux supports solides (filtre, gélose, gel d'électrophorèse), on peut citer notamment les substrats à base d'indoxyl et ses dérivés, et les substrats à base d'hydroxyquinoline ou d'esculétine et leurs dérivés, qui permettent la détection d'activités osidase et estérase. On peut citer également les substrats à base de nitrophénol et nitroaniline et dérivés, permettant de détecter les activités osidases et estérases dans le cas de substrats à base de nitrophénol, et des activités peptidases dans le cas de substrats à base de la nitroaniline. On peut citer enfin les substrats à base de naphtol et naphtylamine et leurs dérivés, qui permettent de détecter les activités osidases et estérases par l'intermédiaire du naphtol, et les activités peptidases par l'intermédiaire de la naphtylamine. Ce substrat peut permettre notamment, mais d'une façon non limitative, la détection d'une activité enzymatique telle que l'activité d'une osidase, peptidase, estérase... Le substrat enzymatique peut également être un substrat naturel dont le produit d'hydrolyse est détecté directement ou indirectement. Comme substrat naturel, on peut notamment citer le Tryptophane pour détecter une activité tryptophanase ou desaminase, un acide aminé cyclique (Tryptophane, Phénylalanine, Histidine, Tyrosine) pour détecter une activité désaminase, le Phosphatidyl Inositol pour détecter une activité phospholipase, ... Lorsque cette activité est une activité métabolique, le substrat est alors un substrat métabolique, telle qu'une source de carbone ou d'azote, couplée à un indicateur produisant une coloration en présence de l'un des produits du métabolisme.
Selon un mode préféré de réalisation de l'invention, ladite première et/ou deuxième activité enzymatique ou métabolique est une activité enzymatique, préférentiellement choisie parmi les activités enzymatiques : beta-glucosidase, desaminase, beta-glucuronidase, beta-galactosidase, alpha-glucosidase, alpha-galactosidase, hexosaminidase, N-acétyl-hexosaminidase, phosphatase, estérase, aminopeptidase.
Par exemple, pour détecter *E. coli,* on utilise préférentiellement l'activité beta-glucuronidase ou ß-galactosidase ou tryptophanase ou desaminase ; pour détecter les *Proteus* on utilise préférentiellement l'activité désaminase, pour détecter les entérocoques, on utilise préférentiellement l'activité beta-glucosidase. Pour les *Candida albicans,* on privilégie l'hexosaminidase, pour les *Listeria monocytogenes* la phospholipase, pour les salmonelles l'estérase, pour les *Pseudomonas aeruginosa* l'estérase ou la ß-alanine aminopeptidase, pour les *Staphylococcus aureus* la phosphatase ou l'alpha-glucosidase.
Par marqueur permettant la différenciation de deux groupes de microorganismes, on entend un composé qui n'a pas les mêmes propriétés sur un premier et sur un deuxième groupe. Ce composé peut être ainsi :
   ● un substrat spécifique
   ● un inhibiteur de mécanisme de résistance, qui permet alors d'inhiber la croissance des organismes développant une résistance particulière, sans discrimination de l'espèce de
   microorganismes

Dans le cas de l'utilisation d'un substrat spécifique, on utilise préférentiellement l'activité beta-glucuronidase, ou beta-galactosidase ou tryptophanaseou desaminase pour détecter E. *coli,* pour détecter les *Proteus* on utilise préférentiellement l'activité désaminase, pour détecter les entérocoques, on utilise préférentiellement l'activité beta-glucosidase. Pour les *Candida albicans,* on privilégie l'hexosaminidase, pour les *Listeria monocytogenes* la phospholipase, pour les salmonelles l'estérase, pour les *Pseudomonas aeruginosa* l'estérase ou la ß-alanine aminopeptidase, pour les *Staphylococcus aureus* la phosphatase ou l'alpha-glucosidase.

Dans le cas de l'utilisation d'un inhibiteur de mécanisme de résistance, on utilise préférentiellement
● l'acide clavulanique, le tazobactam, ou le sulbactam lorsque le premier groupe et/ou le deuxième groupe comprend un mécanisme de résistance induit par l'expression de β-lactamases. La concentration en acide clavulanique dans le milieu est alors préférentiellement comprise entre 0,05 et 32 mg/l, préférentiellement entre 0,1 et 8 mg/l et encore plus préférentiellement entre 0,25 et 6mg/l.
● la cloxacilline ou la dicloxacilline lorsque le premier groupe et/ou le deuxième groupe comprend un mécanisme de résistance induit par l'expression de céphalosporinases

Par taxon, on entend un groupe de microorganismes ayant une unité taxonomique. Un taxon peut être une famille, un genre, un ensemble de genres, une espèce, un ensemble d'espèces, une sous-espèce. A titre indicatif on peut citer les entérobactéries, les *Klebsiella,* les *Escherichia,* les *Enterobacter,* les *Citrobacter,* les *Serratia,* les KESC (*Klebsiella, Enterobacter, Serratia, Citrobacter*), les *Proteeae,* les *Proteus,* les *Morganella,* les *Pseudomonas,* les *Staphylococcus,* les *Streptococcus,* les *Enterococcus,* les *Candida, Escherichia coli, Escherichia coli* O157:H7, *Klebsiella pneumoniae, Citrobacter freundii, Enterococcus faecalis, Enterococcus faecium, Staphylococcus aureus,* les staphylocoques à coagulase négative, *Candida albicans, Candida glabrata, Candida krusei, Candida lusitaniae.*

Par antimicrobien, on entend tout composé susceptible d'empêcher ou de ralentir la croissance d'un microorganisme. Ce composé peut être un antibiotique ou un antifongique. Par antibiotique, on entend tout composé susceptible d'empêcher ou de ralentir la croissance d'une bactérie. A titre indicatif, on peut citer notamment les antibiotiques cefotaxime, ceftazidime, ceftriaxone, cefpodoxime, aztréonam, vancomycine, tobramycine, ciprofloxacine.

Par antifongique, on entend tout composé susceptible d'empêcher ou de ralentir la croissance d'une levure ou d'une moisissure. A titre indicatif, on peut citer notamment l'amphotéricine B, le fluconazole, l'itraconazole, le voriconazole, la cycloheximide.

Selon un mode préféré de réalisation de l'invention, lorsque l'antibiotique est
● la cefotaxime, la concentration en cefotaxime dans le milieu est préférentiellement comprise entre 0,25 et 8 mg/l, préférentiellement entre 1 et 2 mg/l.
● la ceftazidime, la concentration en ceftazidime dans le milieu est préférentiellement comprise entre 0,25 et 8 mg/l, préférentiellement entre 2 et 2,5 mg/l.
● la ceftriaxone, la concentration en ceftriaxone dans le milieu est préférentiellement comprise entre 0,25 et 8 mg/l, préférentiellement entre 1 et 2,5 mg/l.
● la cefpodoxime, la concentration en cefpodoxime dans le milieu est préférentiellement comprise entre 0,1 et 32 mg/l, préférentiellement entre 0,75 et 10 mg/l et encore plus préférentiellement entre 1 et 6 mg/l.
● l'aztréonam, la concentration en aztréonam dans le milieu est préférentiellement comprise entre 0,1 et 8 mg/l, préférentiellement entre 0,75 et 1,5 mg/l.

Selon un mode particulier de réalisation de l'invention, le milieu comprend une combinaison d'au moins deux antibiotiques. Préférentiellement, la combinaison d'au moins deux antibiotiques comprend cefotaxime et ceftazidime.

Quelque soit le mode de réalisation de l'invention, le milieu peut comprendre en outre un colorant. A titre indicatif, on peut citer comme colorant le bleu d'Evans, du rouge neutre, du sang de mouton, du sang de cheval, un opacifiant tel que l'oxyde de Titane, de la nitroaniline, du vert malachite, du vert brillant...

Tous les milieux peuvent comprendre, en outre, afin d'augmenter leur sensibilité
● au moins un antimicrobien actif contre les bactéries gram positif, tel que notamment le linezolide ou la vancomycine.
● au moins un antimicrobien actif contre les levures, tel que notamment le voriconazole ou l'amphotéricine B.
●

L'invention concerne l'utilisation d'un milieu de culture pour distinguer au moins 3 groupes de microorganismes dans un échantillon biologique comprenant :
● un premier groupe de microorganismes, appartenant à un premier taxon de microorganismes et comprenant au moins un mécanisme de résistance à un traitement
● un deuxième groupe de microorganismes, appartenant à un deuxième taxon de microorganismes, différent dudit premier taxon, mais comprenant au moins un mécanisme de résistance à un traitement, identique à celui du premier groupe
● un troisième groupe de microorganismes, non résistants audit traitement
ledit milieu de culture comprenant
a. au moins un premier substrat permettant la détection d'au moins une première activité enzymatique ou métabolique dudit premier groupe de microorganismes
b. au moins un marqueur permettant la différenciation du premier groupe de microorganismes et du deuxième groupe de microorganismes, ledit marqueur étant un substrat permettant la détection d'au moins une activité enzymatique ou métabolique dudit deuxième groupe de microorganismes
c. au moins un antimicrobien, actif sur ledit troisième groupe de microorganismes

Ce mode de réalisation de l'invention permet de distinguer dans un même échantillon, un premier et un deuxième groupe comprenant différentes espèces ou différents taxons de microorganismes, mais chacun des deux groupes étant résistants à un même traitement.

L'invention concerne un milieu de culture comprenant
● un premier substrat permettant la détection d'une activité enzymatique hexosaminidase, préférentiellement le 5-Bromo-4-chloro-3-indolyl-N-acétyl-β-D-glucosaminide à une concentration comprise entre 25 et 500 mg/l, préférentiellement entre 40 et 150 mg/l
● un deuxième substrat permettant la détection d'une activité beta glucosidase, préférentiellement le 6-Chloro-3-indolyl-ß-D-glucoside à une concentration comprise entre 25 et 500 mg/l, préférentiellement entre 40 et 200 mg/l
● un antimicrobien qui est un antifongique qui est préférentiellement l'amphotéricine B (amphoB) à une concentration comprise entre 0,5 et 64 mg/l, préférentiellement entre 1 et 16 mg/l, encore plus préférentiellement entre 1 et 8 mg/l

Lorsque l'antifongique est l'amphotéricine B, ce milieu est préférentiellement utilisé pour distinguer:
● un premier groupe de levures comprenant *Candida albicans* développant une résistance à l'amphoB
● un deuxième groupe de levures, comprenant *Candida tropicalis* et/ou C. *lusitaniae* et ou *C*. *kefyr,* développant une résistance à l'amphoB
● un troisième groupe de levures, non résistantes à l'amphoB

L'invention concerne également un milieu de culture comprenant
● un premier substrat permettant la détection d'une activité enzymatique hexosaminidase, préférentiellement le 5-Bromo-4-chloro-3-indolyl-N-acétyl-β-D-glucosaminide à une concentration comprise entre 25 et 500 mg/l, préférentiellement entre 40 et 150 mg/l
● un deuxième substrat permettant la détection d'une activité phosphatase, préférentiellement le 5-Bromo-6-chloro-3-indolyl-phosphate à une concentration comprise entre 25 et 750 mg/l, préférentiellement entre 40 et 200 mg/l
● un antimicrobien qui est un antifongique qui est préférentiellement de l'amphoB à une concentration comprise entre 0,5 et 64 mg/l, préférentiellement entre 1 et 16 mg/l, encore plus préférentiellement entre 1 et 8 mg/l

Lorsque l'antifongique est l'amphotéricine B, ce milieu est préférentiellement utilisé pour distinguer:
● un premier groupe de levures comprenant *Candida albicans* développant une résistance à l'amphoB
● un deuxième groupe de levures, comprenant *Candida tropicalis* et/ou *C. glabrata* et ou *C. krusei,* développant une résistance à l'amphoB
● un troisième groupe de levures, non résistantes à l'ampho B

L'invention concerne également un milieu de culture comprenant
● un premier substrat permettant la détection de l'activité enzymatique hexosaminidase dudit premier groupe, préférentiellement le 5-Bromo-4-chloro-3-indolyl-N-acétyl-β-D-glucosaminide à une concentration comprise entre 25 et 500 mg/l, préférentiellement entre 40 et 150 mg/l
● un deuxième substrat permettant la détection de l'activité beta glucosidase dudit deuxième groupe, préférentiellement le 6-Chloro-3-indolyl-β-D-glucoside à une concentration comprise entre 25 et 500 mg/l, préférentiellement entre 40 et 200 mg/l
● un antimicrobien qui est un antifongique, préférentiellement du fluconazole à une concentration comprise entre 1 et 256 mg/l, préférentiellement entre 2 et 128 mg/l,
encore plus préférentiellement entre 8 et 64 mg/l

Lorsque l'antifongique est le fluconazole, ce milieu est préférentiellement utilisé pour distinguer:
● un premier groupe de levures comprenant *Candida albicans* développant une résistance au fluconazole
● un deuxième groupe de levures, comprenant *Candida tropicalis* et/ou C. *lusitaniae* et ou *C*. *kefyr,* développant une résistance au fluconazole
● un troisième groupe de levures, non résistantes au fluconazole

L'invention concerne également un milieu de culture comprenant
● un premier substrat permettant la détection d'une activité enzymatique hexosaminidase, préférentiellement le 5-Bromo-4-chloro-3-indolyl-N-acétyl-β-D-glucosaminide à une concentration comprise entre 25 et 500mg/l, préférentiellement entre 40 et 150mg/l
● un deuxième substrat permettant la détection d'une activité phosphatase préférentiellement le 5-Bromo-6-chloro-3-indolyl-phosphate à une concentration comprise entre 25 et 750mg/l, préférentiellement entre 40 et 200mg/l
● un antimicrobien qui est un antifongique qui est préférentiellement du fluconazole à une concentration comprise entre 1 et 256mg/l, préférentiellement entre 2 et 128mg/l,
encore plus préférentiellement entre 8 et 64mg/l

Lorsque l'antifongique est le fluconazole, ce milieu est préférentiellement utilisé pour distinguer:
● un premier groupe de levures comprenant *Candida albicans* développant une résistance au fluconazole
● un deuxième groupe de levures, comprenant *Candida tropicalis* et/ou *C*. *glabrata* et ou *C*. *krusei,* développant une résistance au fluconazole
● un troisième groupe de levures, non résistantes au fluconazole

## Revendications

1. Utilisation d'un milieu de culture pour distinguer au moins trois groupes de microorganismes dans un échantillon biologique, ledit échantillon comprenant :
● un premier groupe de levures, appartenant à un premier taxon de levures et comprenant au moins un mécanisme de résistance à un traitement ;
● un deuxième groupe de levures, appartenant à un deuxième taxon de levures, différent dudit premier taxon, mais comprenant au moins un mécanisme de résistance au même traitement ;
● un troisième groupe de levures non résistantes audit traitement ;
ledit milieu de culture comprenant :
a) au moins un premier substrat permettant la détection d'au moins une première activité enzymatique ou métabolique dudit premier groupe de levures ;
b) au moins un marqueur permettant la différenciation du premier groupe de microorganismes et du deuxième groupe de microorganismes, ledit marqueur étant un substrat permettant la détection d'au moins un activité enzymatique ou métabolique dudit deuxième groupe de levures ;
c) au moins un antimicrobien, actif sur ledit troisième groupe de levures.

2. Milieu de culture comprenant :
● un premier substrat permettant la détection d'une activité enzymatique hexosaminidase ;
● un deuxième substrat permettant la détection d'une activité beta-glucosidase ;
● un antifongique, qui est l'amphotéricine B.

3. Utilisation d'un milieu de culture selon la revendication 2 pour distinguer:
● un premier groupe de levures comprenant *Candida albicans* développant une résistance à l'amphotéricine B;
● un deuxième groupe de levures, comprenant *Candida tropicalis* et/ou *Candida lusitaniae* et/ou *Candida kefyr,* développant une résistance à l'amphotéricine B;
● un troisième groupe de levures, non résistantes à l'amphotéricine B.

4. Milieu de culture comprenant :
● un premier substrat permettant la détection d'une activité enzymatique hexosaminidase ;
● un deuxième substrat permettant la détection d'une activité phosphatase ;
● un antifongique, qui est l'amphotéricine B.

5. Utilisation d'un milieu de culture selon la revendication 4 pour distinguer:
● un premier groupe de levures comprenant *Candida albicans* développant une résistance à l'amphotéricine B ;
● un deuxième groupe de levures, comprenant *Candida tropicalis* et/ou *Candida glabrata* et/ou *Candida krusei,* développant une résistance à l'amphotéricine B;
● un troisième groupe de levures, non résistantes à l'amphotéricine B.

6. Milieu de culture comprenant :
● un premier substrat permettant la détection d'une activité hexosaminidase ;
● un deuxième substrat permettant la détection d'une activité beta-glucosidase ;
● un antifongique, qui est le fluconazole.

7. Utilisation d'un milieu de culture selon la revendication 6 pour distinguer :
● un premier groupe de levures comprenant *Candida albicans* développant une résistance au fluconazole;
● un deuxième groupe de levures, comprenant *Candida tropicalis* et/ou *Candida lusitaniae* et/ou *Candida kefyr,* développant une résistance au fluconazole;
● un troisième groupe de levures, non résistantes au fluconazole.

8. Milieu de culture comprenant :
● un premier substrat permettant la détection d'une activité enzymatique hexosaminidase ;
● un deuxième substrat permettant la détection d'une activité phosphatase ;
● un antifongique, qui est le fluconazole.

9. Utilisation d'un milieu de culture selon la revendication 8, pour distinguer :
● un premier groupe de bactéries comprenant *Candida albicans* développant une résistance au fluconazole;
● un deuxième groupe de levures, comprenant *Candida tropicalis* et/ou *Candida glabrata* et/ou *Candida krusei,* développant une résistance au fluconazole;
● un troisième groupe de levures, non résistantes au fluconazole.
